# EUROPÄISCHE PATENTSCHRIFT

(11) **EP 2 785 420 B1**
(45) Veröffentlichungstag und Bekanntmachung des Hinweises auf die Patenterteilung: **14.03.2018**
(21) Anmeldenummer: 12794246.4
(22) Anmeldetag: 21.11.2012
(51) Int. Cl.: A61Q 19/06, A61K 8/65

(54) **KOSMETISCHE VERWENDUNG VON KOLLAGENHYDROLYSAT**
COSMETIC USE OF COLLAGEN HYDROLYSATE
TRAITEMENT COSMÉTIQUE DE L'HYDROLYSAT DE COLLAGÈNE

(30) Priorität: 29.11.2011 DE 102011055800; 07.03.2012 DE 102012101911
(43) Veröffentlichungstag der Anmeldung: 08.10.2014
(73) Patentinhaber: Gelita AG, 69412 Eberbach (DE)
(72) Erfinder: FRECH, Hans-Ulrich, 69469 Weinheim (DE); OESSER, Steffen, 24960 Glücksburg (DE); HAUSMANNS, Stephan, 69126 Heidelberg (DE)
(74) Vertreter: Hoeger, Stellrecht & Partner Patentanwälte mbB
(86) Internationale Anmeldenummer: PCT/EP2012/073180
(87) Internationale Veröffentlichungsnummer: WO 2013/079373

(56) Entgegenhaltungen:
- WO-A1-2011/011808
- anonymous: "Collagen peptides for a healthy lifestyle - Peptan", , 1. Februar 2009 (2009-02-01), Seiten 1-8, XP002692958, Gefunden im Internet: URL:www.peptan.com/en/peptan-for.../asset/ ...peptan.../download.file [gefunden am 2013-02-25]
- VIVIAN ZAGUE: "A new view concerning the effects of collagen hydrolysate intake on skin properties", ARCHIVES OF DERMATOLOGICAL RESEARCH ; FOUNDED IN 1869 AS ARCHIV FÜR DERMATOLOGIE UND SYPHILIS, SPRINGER, BERLIN, DE, Bd. 300, Nr. 9, 11. September 2008 (2008-09-11), Seiten 479-483, XP019657199, ISSN: 1432-069X, DOI: 10.1007/S00403-008-0888-4
- JIANG LIANG ET AL: "The Protective Effects of Long-Term Oral Administration of Marine Collagen Hydrolysate from Chum Salmon on Collagen Matrix Homeostasis in the Chronological Aged Skin of Sprague-Dawley Male Rats", JOURNAL OF FOOD SCIENCE, Bd. 75, Nr. 8, 1. Oktober 2010 (2010-10-01), Seiten H230-H238, XP055054559, ISSN: 0022-1147, DOI: 10.1111/j.1750-3841.2010.01782.x
- ZAGUE VIVIAN ET AL: "Collagen Hydrolysate Intake Increases Skin Collagen Expression and Suppresses Matrix Metalloproteinase 2 Activity", JOURNAL OF MEDICINAL FOOD, MARY ANN LIEBERT, LARCHMONT, NY, US, Bd. 14, Nr. 6, 1. Juni 2011 (2011-06-01), Seiten 618-624, XP009167423, ISSN: 1096-620X, DOI: 10.1089/JMF.2010.0085 [gefunden am 2011-04-11]
- DORIS HEXSEL ET AL: "Cosmeceuticals for Cellulite", SEMINARS IN CUTANEOUS MEDICINE AND SURGERY, Bd. 30, Nr. 3, 1. November 2011 (2011-11-01), Seiten 167-170, XP028295368, ISSN: 1085-5629, DOI: 10.1016/J.SDER.2011.06.005 [gefunden am 2011-07-12]

## Beschreibung

Die vorliegende Erfindung betrifft Kollagenhydrolysat und dessen Verwendung zur Behandlung und/oder Vorbeugung von Cellulite.
Bei Cellulite handelt es sich um eine unerwünschte Veränderung der Eigenschaften der Haut, die sich äußerlich durch die Bildung von Dellen an der Hautoberfläche bemerkbar macht. Betroffen sind fast ausschließlich Frauen, von diesen jedoch mit zunehmendem Alter bis zu 80 bis 98%. Cellulite tritt vor allem in Bereichen mit ausgeprägtem subkutanem Fettgewebe auf, d.h. an der Hüfte, dem Gesäß, dem Bauch, den Oberschenkeln und den Oberarmen.
Als Ursache für Cellulite gelten nach dem Stand der Forschung bestimmte Veränderungen in der Dermis und dem subkutanem Bindegewebe, insbesondere eine Verkürzung der aus Kollagenfasern gebildeten Septen, die die retikuläre Dermis mit den unter dem subkutanen Fettgewebe liegenden Muskeln verbinden. Dies führt insgesamt zu einer Verringerung der Elastizität der Haut. Bekannte Therapien gegen Cellulite umfassen insbesondere physikalische Methoden wie Lymphdrainage, Ultraschall oder Vakuum, diese bringen jedoch in der Regel keinen oder zumindest keinen dauerhaften Erfolg. Auch die topische Anwendung von Kosmetika wie Cremes oder Salben ermöglicht keine ursächliche Behandlung der Cellulite, da die oberen Hautschichten der Epidermis an dem Phänomen nicht direkt beteiligt sind.
Es ist bereits bekannt, dass durch die orale Aufnahme von Kollagenhydrolysat vorteilhafte Effekte in Bezug auf die Gesundheit der Haut beim Menschen erzielt werden können (siehe V. Zague: "A new view concerning the effects of collagen hydrolysate intake on skin properties" in Arch. Dermatol. Res. 2008 (9) 479). Aufgrund der Resorptionsfähigkeit entsprechend niedermolekularer Kollagenpeptide und der guten Durchblutung der Haut erfolgt dort in besonderem Maße eine Akkumulation von oral aufgenommenem Kollagenhydrolysat, wobei die Konzentration im Zeitraum von etwa 12 bis 24 Stunden nach der Aufnahme am höchsten ist (siehe M. Watanabe-Kamiyama et al.: "Absorption and effectiveness of orally administered low molecular weight collagen hydrolysate in rats" in J. Agric. Food Chem. 2010 (58) 835).
In der Broschüre "Peptan - Das ideale Protein für einen gesunden Lebensstil", veröffentlicht von dem Unternehmen Rousselot im Februar 2009, wird Kollagenhydrolysat als Nahrungsergänzung beworben, u. a. zur Verbesserung der Festigkeit und Elastizität der Haut.
In einem Artikel von J. Liang et al. in Journal of Food Science 2010 (75) H230-H238 wird in einem Tierversuch mit Ratten gezeigt, dass marines Kollagenhydrolysat aus Lachshaut einen Einfluss auf die Homöostase der Kollagenmatrix in gealterter Haut aufweist.
In einem Artikel von V. Zague et al. in Journal of Medicinal Food 2011 (14) 618-624 wird ebenfalls in einem Tierversuch mit Ratten gezeigt, dass die Aufnahme von Kollagenhydrolysat zu einem Anstieg der Kollagenexpression in der Haut führt und die Aktivität der Metalloproteinase 2 unterdrückt.
In einem Artikel von D. Hexsel et al. in Seminars on Cutaneous Medicine and Surgery 2011 (30) 167-170 werden verschiedene Wirkstoffe beschrieben, die als "Cosmeceuticals" zur Behandlung von Cellulite vorgeschlagen wurden, wobei es für deren Wirksamkeit kaum klinische Belege gibt.
Vor diesem Hintergrund schlägt die vorliegende Erfindung einen neuen Ansatz für die ursächliche Behandlung und/oder Vorbeugung von Cellulite vor, wobei dieser Ansatz in der Verwendung von Kollagenhydrolysat besteht. Die Erfindung ist die, die in den Ansprüchen enthalten ist. Ein Aspekt der Erfindung betrifft somit die kosmetische Verwendung von Kollagenhydrolysat zur Behandlung und/oder Vorbeugung von Cellulite, wobei das Kollagenhydrolysat ein mittleres Molekulargewicht von 1.700 bis 2.300 Da aufweist, wobei mindestens 45 Gew.% des Kollagenhydrolysats ein Molekulargewicht von weniger als 1.500 Da aufweisen, und wobei das Kollagenhydrolysat für eine oral Verabreichung vorgesehen ist.

Tatsächlich konnte in einer klinischen Studie gezeigt werden, dass in Folge der Verabreichung von Kollagenhydrolysat die Hautelastizität messbar zunimmt. Dieser Effekt ist bei Frauen im Alter von über 50 Jahren besonders ausgeprägt. Eine höhere Hautelastizität bedeutet eine geringere Ausprägung von Cellulite.

Des Weiteren belegen verschiedene Versuche *in vitro,* dass die Synthese von extrazellulären Matrixproteinen des dermalen Bindegewebes durch Kollagenhydrolysat stimuliert wird. Diese von den Hautzellen (dermalen Fibroblasten) gebildeten Proteine umfassen Kollagen (insbesondere vom Typ I), Elastin und Proteoglykane (wie Biglykan, Versican und Decorin). Die Synthese dieser Proteine in ausreichender Menge ist entscheidend für den Aufbau bzw. die Regeneration der extrazellulären Matrix der Haut, die wiederum wesentlich bestimmend ist für die Eigenschaften der Dermis wie Elastizität, Spannkraft und Feuchtigkeitshaushalt.

Das im Rahmen der Erfindung verwendete Kollagenhydrolysat weist ein relativ geringes Molekulargewicht auf. Dabei weisen mindestens 45 Gew.% des Kollagenhydrolysats ein Molekulargewicht von weniger als 1.500 Da auf. Es hat sich gezeigt, dass durch solche besonders niedermolekularen Anteile deutlichere Effekte erzielt werden können. Die Molekulargewichtsverteilung des Kollagenhydrolysats, die den entsprechenden Grenzwerten zu Grunde liegt, kann z.B. mittels einer Gelpermeationschromatographie unter Verwendung eines Kalibrierungsstandards aus definierten Kollagenfragmenten sehr genau und reproduzierbar bestimmt werden.

Das mittlere Molekulargewicht (Gewichtsmittel M_{W}) des erfindungsgemäß verwendeten Kollagenhydrolysats liegt im Bereich von ca. 1.700 bis ca. 2.300 Da.

Bei einer bevorzugten Ausführungsform der Anmeldung umfasst das Kollagenhydrolysat mindestens vier charakteristische Peptide mit einem Molekulargewicht zwischen 600 und 1.200 Da. Kollagenhydrolysate enthalten Peptide mit unterschiedlichen Kettenlängen bzw. Molekulargewichten, die bei der Spaltung der Proteinketten des Kollagens entstehen, wobei sich die Molekulargewichtsverteilungen dieser Peptide in Abhängigkeit von den Herstellungsbedingungen des Hydrolysats deutlich unterscheiden können. Es hat sich überraschenderweise gezeigt, dass sich ein Kollagenhydrolysat mit den vorstehend genannten Eigenschaften besonders vorteilhaft auf die Synthese von Matrixproteinen auswirkt, d.h. deutlich bessere Ergebnisse zeigt als Kollagenhydrolysate, die die charakteristischen Peptide nicht enthalten.
Das Vorhandensein der charakteristischen Peptide des Kollagenhydrolysats kann insbesondere mittels MALDI-Massenspektroskopie bestimmt werden, wobei die charakteristischen Peptide im Massenspektrum als Peaks auftreten. Bevorzugt weisen die mindestens vier charakteristischen Peptide in einer mittels MALDI-Massenspektroskopie bestimmten Molekulargewichtsverteilung eine mindestens doppelte Intensität, weiter bevorzugt eine mindestens vierfache Intensität im Vergleich zu ihrer Umgebung auf.
Bei einer bevorzugten Ausführungsform der Erfindung umfasst das Kollagenhydrolysat ein Peptid zwischen 620 und 690 Da, ein Peptid zwischen 790 und 860 Da, ein Peptid zwischen 980 und 1.050 Da und ein Peptid zwischen 1.175 und 1.245 Da auf. Das Kollagenhydrolysat kann zusätzlich auch charakteristische Peptide zwischen 1.500 und 3.500 Da aufweisen.

Bevorzugt weist das Kollagenhydrolysat einen Anteil an Hydroxyprolin von 12 Gew.% oder mehr auf. Die durch post-translationale Hydroxylierung von Prolin gebildete Aminosäure Hydroxyprolin kommt ausschließlich in Kollagen vor, sodass ein hoher Anteil an Hydroxyprolin im Kollagenhydrolysat ein Maß für die weitgehende Abwesenheit von anderen Bindegewebsproteinen (z.B. Elastin und Proteoglykane) darstellt, deren Fragmente in Abhängigkeit vom Herstellungsverfahren in gewissen Mengen ebenfalls in Kollagenhydrolysaten enthalten sein können.

Günstig ist es, wenn das Kollagenhydrolysat durch enzymatische Hydrolyse von Gelatine hergestellt ist. Gelatine umfasst denaturiertes Kollagen und wird mittels verschiedener, dem Fachmann bekannter Verfahren aus dem Bindegewebe oder aus den Knochen verschiedener Tierarten gewonnen. Im Rahmen der vorliegenden Erfindung wird die als Ausgangsmaterial für das Kollagenhydrolysat eingesetzte Gelatine vorzugsweise aus der Haut von Säugetieren, insbesondere von Schweinen oder Rindern, gewonnen, wobei aber auch die Verwendung von Gelatine aus Geflügel nicht ausgeschlossen ist. Porcine Gelatine ist als Ausgangsmaterial besonders bevorzugt, insbesondere Schweineschwartengelatine.

Die enzymatische Hydrolyse der Gelatine erfolgt in der Regel durch mindestens eine Endoprotease, wobei es im Rahmen der Erfindung bevorzugt ist, mehrere Endoproteasen (d.h. mindestens zwei verschiedene Endoproteasen) einzusetzen, um dadurch das Aminosäureprofil des resultierenden Kollagenhydrolysats entsprechend zu beeinflussen und die positive Wirkung des Hydrolysats zu erhöhen.

Gemäß einer bevorzugten Ausführungsform der Erfindung ist das Kollagenhydrolysat durch die nacheinander folgende Einwirkung von mindestens zwei Endoproteasen mit einer unterschiedlichen Spezifität, insbesondere von mindestens zwei verschiedenen Metalloproteasen und/oder Serinproteasen, hergestellt, d.h. von Proteasen, die die Aminosäuresequenz der Kollagenmoleküle jeweils vor bzw. hinter bestimmten Aminosäuren spalten. Günstigerweise handelt es sich bei den Metalloproteasen und/oder Serinproteasen um Enzyme aus den Mikroorganismen *Bacillus subtilis, Bacillus licheniformis, Bacillus amyloliquefaciens, Aspergillus oryzae* und *Aspergillus melleus.*

Durch die Auswahl geeigneter Endoproteasen kann nicht nur die charakteristische Molekulargewichtsverteilung des Kollagenhydrolysats erhalten werden, sondern es wird auch die Art der Aminosäuren an den Termini der in dem Hydrolysat enthaltenen Peptide beeinflusst. In dieser Hinsicht ist es z.B. bevorzugt, wenn mindestens 50% der N-terminalen Aminosäuren des Kollagenhydrolysats hydrophobe Aminosäuren sind, insbesondere Alanin, Leucin und Isoleucin.

Gemäß der Erfindung ist das Kollagenhydrolysat für eine enterale Anwendung vorgesehen, nämlich in Form einer oralen Aufnahme. Bei oraler Aufnahme ergibt sich ein effektiverer Transport des Kollagenhydrolysats über den Blutkreislauf zum Wirkungsort, d.h. insbesondere zu den dermalen Fibroblasten, als bei einer topischen Anwendung. Zudem ist diese Anwendungsform für den Benutzer in der Regel mit einem wesentlich geringeren Aufwand verbunden.

Da Kollagenhydrolysat aus lebensmittelrechtlich zugelassenen Rohstoffen gewonnen wird, kann es im Rahmen der vorliegenden Erfindung zur Behandlung und/oder Vorbeugung von Cellulite bevorzugt als Nahrungsergänzungsmittel eingesetzt werden. Solche Nahrungsergänzungsmittel können als "Nutraceuticals" oder "Nutricosmetics" bezeichnet werden.

Das Nahrungsergänzungsmittel kann in nahezu beliebiger Form angeboten werden, z.B. in Form von Tabletten, Kapseln, Dragees, Pastillen, Sachets oder auch eines Gels oder einer Lösung (z.B. in Einzelampullen oder in Getränken).

Das Kollagenhydrolysat kann alternativ auch in einem Nahrungs- oder Genussmittel enthalten sein, z.B. in Süßwaren oder in einem Instantpulver zur Zubereitung von Getränken. Das Hydrolysat kann auf diese Weise vom Benutzer ohne zusätzlichen Aufwand im Rahmen der normalen Ernährung aufgenommen werden (so genanntes "Functional Ford"). In diesem Zusammenhang ist es besonders vorteilhaft, wenn das Kollagenhydrolysat im Wesentlichen geschmacksneutral ist.

Günstig ist es, wenn eine täglich Aufnahme von ca. 1,5 bis 5 g, bevorzugt ca. 2 bis 3 g, weiter bevorzugt ca. 2,3 bis 2,7 g des Kollagenhydrolysats vorgesehen ist. Es hat sich gezeigt, dass durch die orale Aufnahme dieser Menge an Hydrolysat bereits ein deutlicher Effekt erzielt werden kann, der sich durch eine Erhöhung der Tagesdosis nicht mehr wesentlich steigern lässt.

Das Kollagenhydrolysat kann im Rahmen der erfindungsgemäßen Verwendung mit weiteren Wirkstoffen kombiniert werden, die eine vorteilhafte Wirkung auf die Gesundheit und insbesondere auf die Gesundheit der Haut haben, unter anderem mit Wirkstoffen mit einer antioxidativen Wirkung. Solche Wirkstoffe sind bevorzugt ausgewählt aus Vitaminen, insbesondere Vitamin C und E, Mineralien, Omega-3-Fettsäuren, Omega-6-Fettsäuren, Omega-9-Fettsäuren, Biotin, Lutein, Lycopin, Coffein, Glucosamin, Chondroitin, Hyaluronsäure, Folsäure, Aminosäuren, Ubichinon-10, Superoxid-Dismutase sowie Pflanzenextrakten aus Hagebutten, Zitronenverbene oder Grüntee.

Bei einer bevorzugten Ausführungsform der Erfindung ist die Verabreichung des Kollagenhydrolysats zur Behandlung und/oder Vorbeugung von Cellulite insbesondere an Frauen im Alter von über 50 Jahren vorgesehen, die sich meistens in der Postmenopause befinden. Bei dieser Altersgruppe, die im Allgemeinen sehr stark von Cellulite betroffen ist, sind die Effekte besonders deutlich, wie durch die weiter unten beschriebene klinische Studie gezeigt wurde.

Des Weiteren betrifft die Erfindung auch die Verwendung von Kollagenhydrolysat zur kosmetischen Behandlung und/oder Vorbeugung von Cellulite oder von Dehnungsstreifen, die insbesondere in Form von so genannten Schwangerschaftsstreifen (*Striae gravidarum*) bei schwangeren Frauen auftreten, wobei das Kollagenhydrolysat ein mittleres Molekulargewicht von 1.700 bis 2.300 Da aufweist, und wobei mindestens 45 Gew.% des Kollagenhydrolysats ein Molekulargewicht von weniger als 1.500 Da aufweisen. Die Ursache für diese Streifen sind feine Risse im subkutanen Bindegewebe auf Grund der erheblichen Hautdehnung. Ähnlich wie bei der Cellulite kann auch dem Auftreten solcher Risse durch eine Erhöhung der Hautelastizität mittels Verabreichung von Kollagenhydrolysat entgegengewirkt werden.

Ein weiterer Aspekt der Erfindung betrifft die Verwendung von Kollagenhydrolysat zur Behandlung und/oder Vorbeugung von lokalen Schädigungen der Haut als Folge des Wundliegens (*Dekubitus*), wie z.B. die Entstehung von Druckgeschwüren. In diesem Fall wird die Haut einer Druckbelastung von außen ausgesetzt, deren negative Folgen sich ebenfalls durch eine Erhöhung der Hautelastizität abmildern lassen.

Diese und weitere Vorteile der Erfindung werden anhand der nachfolgenden Beispiele unter Bezugnahme auf die Figuren näher beschrieben. Es zeigen im Einzelnen:
- Figuren 1A bis 1C:: Diagramme betreffend die Stimulation der Synthese von Typ-I-Kollagen, Biglykan bzw. Versican;
- Figuren 2A und 2B:: Diagramme betreffend die Erhöhung der Hautfeuchtigkeit bei haarlosen Mäusen;
- Figur 3:: ein Diagramm betreffend die Stimulation der Synthese von CE-Proteinen;
- Figur 4A bis 4C:: MALDI-Massenspektren verschiedener Kollagenhydrolysate; und
- Figur 5A und 5B:: Diagramme betreffend die Stimulation der Synthese von Typ-I-Kollagen, Decorin und Versican.

### Beispiele

### 1. Herstellung und Eigenschaften des Kollagenhydrolysats

Zur Herstellung eines Kollagenhydrolysats für die erfindungsgemäße Verwendung wird als Ausgangsmaterial eine wässrige Lösung einer Schweineschwartengelatine (Typ A, 200 bis 250 g Bloom) mit einer Konzentration von 20 bis 40 Gew.% (Trockensubstanz) eingesetzt. Die Gelatine wird durch die nacheinander folgende Einwirkung von zwei verschiedenen Endoproteasen mikrobiellen Ursprungs bei 50 bis 60 °C während 120 bis 180 min enzymatisch hydrolysiert, wobei als erstes Enzym eine Endoprotease aus *Bacillus subtilis* oder aus *Bacillus amyloliquefaciens* verwendet wird und als zweites Enzym eine Endoprotease aus *Bacillus licheniformis.* Anschließend werden die Enzyme thermisch inaktiviert und die Lösung sprühgetrocknet.

Die Molekulargewichtsverteilung des resultierenden Kollagenhydrolysats kann mittels Gelpermeationschromatographie unter Verwendung der folgenden Parameter bestimmt werden:

| | |
|---|---|
| Stationäre Phase: | TSK 2000 SW XL (Tosoh Bioscience GmbH) |
| Mobile Phase: | 0,4 mol/l Natriumdihydrogenphosphat pH 5,3 |
| Flussrate: | 0,5 ml/min |
| Kalibrierungsstandard: | definierte Kollagen-Typ-I-Fragmente (FILK, Freiberg) |
| Detektion: | UV-Detektor Knauer K-2501 bei 214 nm |

Die Bestimmung ergab eine Molekulargewichtsverteilung für dieses Kollagenhydrolysats (im Folgenden als niedermolekulares Hydrolysat bezeichnet) gemäß der nachfolgenden Tabelle 1. Zum Vergleich ist in der Tabelle 1 auch die Molekulargewichtsverteilung eines kommerziell erhältlichen Kollagenhydrolysats angegeben, die mit derselben Methode bestimmt wurde (im Folgenden als hochmolekulares Hydrolysat bezeichnet):

**Tabelle 1**

| MW-Bereich | niedermol. Hydrolysat | hochmol. Hydrolysat |
|---|---|---|
| > 7.500 Da | < 5 Gew.% | < 10 Gew.% |
| 3.500 - 7.500 Da | ca. 12-18 Gew.% | ca. 25-35 Gew.% |
| 1.500 - 3.500 Da | ca. 25-31 Gew.% | ca. 29-35 Gew.% |
| 500 - 1.500 Da | ca. 40-46 Gew.% | ca. 24-30 Gew.% |
| < 500 Da | ca. 5-10 Gew.% | ca. 2-5 Gew.% |

Der Hydroxyprolingehalt dieses niedermolekularen Hydrolysats beträgt ca. 12 bis 13 Gew.% und kann nach Oxidation mit Chloramin-T und Umsetzung mit p-Dimethylamino-benzaldehyd photometrisch bestimmt werden. Mehr als 50% der N-terminalen Aminosäuren des Hydrolysats sind hydrophobe Aminosäuren, insbesondere Alanin, Leucin und Isoleucin.

### 2. Klinische Studie zur Wirksamkeit des Kollagenhydrolysats bei Cellulite

In einer doppelblinden, randomisierten, Placebo-kontrollierten Studie wurde die Wirksamkeit des gemäß Beispiel 1 hergestellten niedermolekularen Kollagenhydrolysats bei der Behandlung und/oder Vorbeugung von Cellulite untersucht. Bei den Studienteilnehmern handelte es sich um 69 gesunde Frauen im Alter von 35,3 bis 55,4 Jahren, die in drei Gruppen mit jeweils 23 Probanden eingeteilt wurden. 68 Probanden beendeten die Studie erfolgreich.

Ab sechs Wochen vor dem Beginn der Studie durften keine dermatologischen Therapeutika angewendet werden, zudem durften die Probanden vor und während der Studie ihre Lebens- und Ernährungsgewohnheiten nicht ändern, keine zusätzlichen Nahrungsergänzungsmittel oder Vitaminpräparate einnehmen und ihre Haut keiner intensiven UV-Strahlung aussetzen. An den volaren Seiten der Unterarme, wo die Auswirkungen des Kollagenhydrolysats auf die Hauteigenschaften untersucht werden sollten, durften keine Kosmetika eingesetzt werden.

Von den drei Gruppen erhielt die erste während eines Zeitraums von acht Wochen täglich 2,5 g Kollagenhydrolysat (jeweils morgens), die zweite täglich 5 g Kollagenhydrolysat (jeweils 2,5 g morgens und mittags) und die dritte ein Placebo. Zur oralen Aufnahme konnte das Hydrolysat in Wasser oder einem kalten Getränk (mit Ausnahme von Milch) aufgelöst werden.

Vor der ersten Einnahme, nach vier Wochen und nach acht Wochen wurden folgende Parameter der Haut an der volaren Seite des linken Oberarms der Probanden gemessen:
- Hautelastizität mit einem Cutometer® SEM 575 (jeweils Mittelwert aus drei Messungen)
- transepidermaler Wasserverlust (TEWL) mit einem DermaLab®-Gerät (jeweils Mittelwert aus drei Messungen)
- Hautfeuchtigkeit mit einem Corneometer® CM 825 (jeweils Mittelwert aus zehn Messungen)

Alle Messungen wurden nach 30minütigen Akklimatisierung in einem klimatisierten Raum bei einer Temperatur von 21,5 °C (± 1 °C) und einer relativen Luftfeuchtigkeit von 50% (± 5%) durchgeführt.

Alle drei Parameter waren bei den mit Kollagenhydrolysat behandelten Gruppen sowohl nach vier als auch nach acht Wochen signifikant erhöht. Die nach acht Wochen gemessenen Werte sind in der nachfolgenden Tabelle 2 angegeben, und zwar als prozentuale Steigerung gegenüber der mit dem Placebo behandelten Gruppe:

**Tabelle 2**

| Parameter | 2,5 g Hydrolysat pro Tag | 5 g Hydrolysat pro Tag |
|---|---|---|
| Hautelastizität | ca. 7% | ca. 9% |
| TEWL | ca. 11% | ca. 14% |
| Hautfeuchtigkeit | ca. 6% | ca. 7% |

Insbesondere die Erhöhung der Hautelastizität belegt die Wirksamkeit der oralen Verabreichung von Kollagenhydrolysat zur Behandlung und/oder Vorbeugung von Cellulite. Die Verbesserung der TEWL und der Hautfeuchte stellen weitere vorteilhafte Effekte des Hydrolysats auf die Hautgesundheit dar und führen insbesondere zu einer Erhöhung der epidermalen Barrierefunktion.

Bei einer nach Altersgruppen differenzierten Betrachtung der Erhöhung der Hautelastizität ergab sich das in der Tabelle 3 dargestellte Ergebnis, wobei Frauen unter 50 Jahren (Durchschnittsalter 44,1 Jahre) Frauen über 50 Jahren (Durchschnittsalter 53,0 Jahre) gegenübergestellt wurden:

**Tabelle 3**

| Hautelastizität | 2,5 g Hydrolysat pro Tag | 5 g Hydrolysat pro Tag |
|---|---|---|
| Frauen unter 50 | ca. 3% | ca. 5% |
| Frauen über 50 | ca. 14% | ca. 15% |

Auffallend ist die besonders deutliche Verbesserung der Hautelastizität bei Frauen im Alter von über 50 Jahren, die somit eine bevorzugte Zielgruppe für die erfindungsgemäße Verwendung von Kollagenhydrolysat darstellen.

Die Hautelastizität wurde nochmals vier Wochen nach Ende des achtwöchigen Verabreichungszeitraums gemessen. Dabei waren noch etwa 92 bis 98% der nach acht Wochen gemessenen Steigerungen erhalten, was auf eine länger anhaltende Wirkung des Kollagenhydrolysats hindeutet.

### 3. Stimulation der Synthese von extrazellulären Matrixproteinen in vitro

Die Stimulation der Synthese von Kollagen (Typ I) sowie der Proteoglykane Biglykan und Versican wurde *in vitro* an humanen dermalen Fibroblasten (Hautzellen) untersucht. Hierfür wurden die Zellen für 24 Stunden mit jeweils 0,5 mg/ml des niedermolekularen bzw. des hochmolekularen Hydrolysats inkubiert und anschließend die Expression von Kollagen-RNA, Biglykan-RNA und Versican-RNA mittels Realtime-PCR bestimmt und semiquantitativ (bezogen auf eine Kontrolle ohne Hydrolysat) ausgewertet.

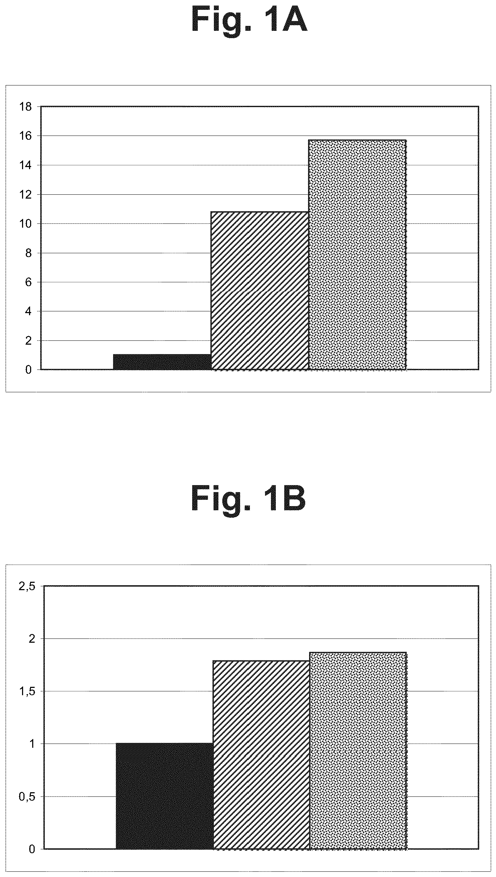

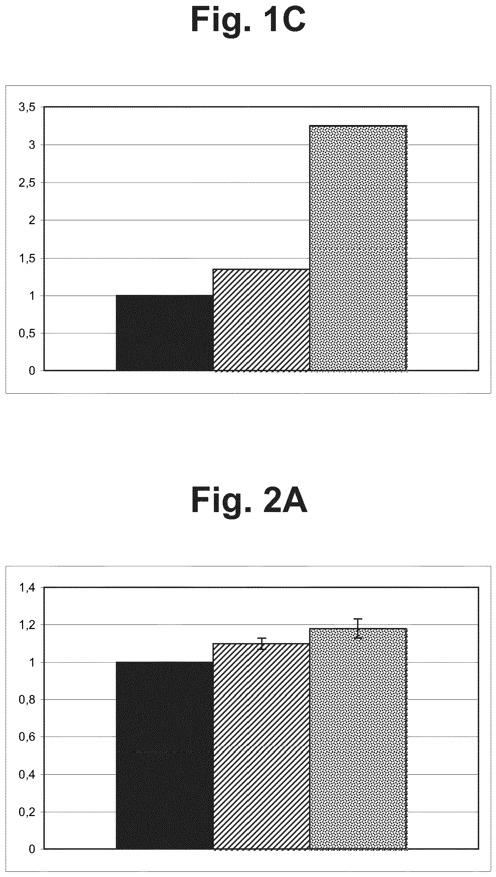

Die Ergebnisse sind als Säulendiagramme für Typ-I-Kollagen in der Figur 1A, für Biglykan in der Figur 1B und für Versican in der Figur 1C dargestellt, wobei die Diagramme jeweils den Mittelwert aus mindestens 18 Messungen zeigen. Auf der Abszisse ist die RNA-Expression relativ zur Kontrolle (=1) aufgetragen. Die linke ausgefüllte Säule steht jeweils für die Kontrolle, die mittlere schraffierte Säule für das hochmolekulare Hydrolysat und die rechte gepunktete Säule für das niedermolekulare Hydrolysat.

Es zeigt sich, dass die Synthese aller drei Matrixproteine durch beide Kollagenhydrolysate stimuliert wird, wobei der positive Effekt des niedermolekularen Hydrolysats jeweils stärker ausgeprägt ist als derjenige des hochmolekularen Hydrolysats. Beim Kollagen, welches neben Elastin hauptverantwortlich für die Spannkraft und Elastizität der Haut ist, sowie beim Versican, das für den Feuchtigkeitshaushalt der Haut eine wichtige Rolle spielt, fällt der verstärkte Effekt des niedermolekularen Hydrolysats besonders deutlich aus.

Diese stimulierenden Eigenschaften des Kollagenhydrolysats auf die verschiedenen Matrixproteine bieten neben der erfindungsgemäßen Behandlung und/oder Vorbeugung von Cellulite auch einen Ansatzpunkt bei Erkrankungen wie z.B. Schuppenflechte (Psoriasis), bei denen die natürliche Funktionalität der Haut beeinträchtigt ist.

### 4. Erhöhung des Feuchtigkeitsgehalts der Haut im Tierversuch

Die Beeinflussung der Hautfeuchtigkeit durch Kollagenhydrolysat wurde an haarlosen Mäusen direkt untersucht. Haarlose Mäuse stellen ein etabliertes Modellsystem dar, das bei dermatologischen Fragestellungen häufig eingesetzt wird, und die daraus gewonnenen Erkenntnisse sind prinzipiell auf die menschliche Haut übertragbar (siehe z.B. T. Fujimura et al.; J. Dermatol. Sci. 2000 (24) 105-111 und Y. Nishimori et al.; J. Invest. Dermatol. 2001 (117) 1458-1463).

Die Tiere wurden während eines Zeitraums von drei Wochen täglich mit 150 µg Kollagenhydrolysat pro kg Körpergewicht gefüttert, die Kontrollgruppe erhielt stattdessen BSA. Gleichzeitig erhielten alle Tiere eine wöchentliche UV-B-Strahlungsdosis von 18 mJ/cm² Hautoberfläche, wodurch die Hautfeuchtigkeit negativ beeinflusst wird.

Der Feuchtigkeitsgehalt der Haut wurde nach einer Woche und nach drei Wochen mit einem Corneometer CM 825 (Hersteller: Courage & Khazaka) gemessen. Das Messprinzip beruht hierbei auf der Änderung der Kapazität eines Messkondensators durch die Dielektrizitätskonstante des in den oberen Hautschichten gebundenen Wassers, die sich von den Dielektrizitätskonstanten der meisten anderen Stoffe deutlich unterscheidet.

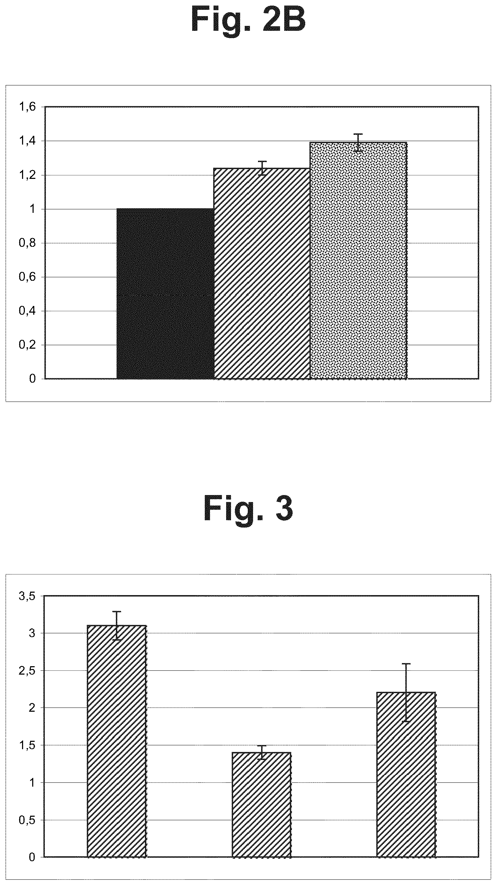

Die Ergebnisse sind als Säulendiagramme für die Messung nach einer Woche in der Figur 2A und für die Messung nach drei Wochen in der Figur 2B dargestellt, wobei die Diagramme jeweils den Mittelwert und den Standardfehler aus 7 Messungen zeigen. Auf der Abszisse ist die Hautfeuchtigkeit relativ zur Kontrolle (=1) aufgetragen. Die linke ausgefüllte Säule steht jeweils für die Kontrolle, die mittlere schraffierte Säule für das hochmolekulare Hydrolysat und die rechte gepunktete Säule für das niedermolekulare Hydrolysat.

Es zeigt sich, dass die Erhöhung der Hautfeuchtigkeit durch das niedermolekulare Hydrolysat sowohl nach einer Woche als auch nach drei Wochen jeweils stärker ist als durch das hochmolekulare Hydrolysat.

### 5. Stimulation der Synthese von CE-Proteinen in vitro

So genannte "Cornified Envelope"-Proteine spielen eine wichtige Rolle für die Barrierefunktion der Haut gegen das Eindringen von pathogenen Keimen und toxischen Substanzen. Die Synthese der CE-Proteine Involucrin, Loricrin und Filaggrin wurde bei haarlosen Mäusen bestimmt, die zuvor fünf Wochen mit täglich 150 µg Kollagenhydrolysat pro kg Körpergewicht gefüttert wurden (wie oben beschrieben). Die Quantifizierung der Proteine relativ zu einer Kontrollgruppe (Fütterung mit BSA) erfolgte mittels SDS-Polyacrylamid-Gelelektrophorese und Western-Blot mit spezifischen Antikörpern nach Extraktion der Proteine aus der Haut.

Die Ergebnisse sind als Säulendiagramm in der Figur 3 dargestellt, wobei das Diagramm jeweils den Mittelwert und den Standardfehler aus 7 Messungen zeigt. Auf der Abszisse ist die Menge der CE-Proteine nach Fütterung mit dem niedermolekularen Hydrolysat relativ zur Kontrolle (=1) aufgetragen. Die linke Säule steht für Involucrin, die mittlere Säule für Loricrin und die rechte Säule für Filaggrin.

Es zeigt sich, das die Synthese aller drei untersuchten CE-Proteine durch die orale Aufnahme von Kollagenhydrolysat stimuliert wird, im Fall von Involucrin sogar um mehr als das Dreifache.

### 6. Analyse der Molekulargewichtsverteilung mittels MALDI-MS

Das gemäß Beispiel 1 hergestellte, niedermolekulare Kollagenhydrolysat, das ein mittleres Molekulargewicht von ca. 2.000 Da aufweist (im folgenden Hydrolysat A), wurde zwei kommerziell erhältlichen Kollagenhydrolysaten mit einem mittleren Molekulargewicht von ca. 2.100 Da (im Folgenden Hydrolysat B) und ca. 2.900 Da (im Folgenden Hydrolysat C) gegenübergestellt.

Die genauen Molekulargewichtsverteilungen dieser drei Hydrolysate wurden mittels MALDI-Massenspektroskopie (MALDI-MS) analysiert. Hierzu wurden die Proben in 0,1%iger Trifluoressigsäure auf eine Endkonzentration von 10 µg/µl eingestellt und anschließend unter Verwendung von µC₁₈-Material gereinigt. Die Proben wurden mit einer HCCA-Matrix auf ein MALDI-Target präpariert und die Massenspektren unter Verwendung eines Ultraflex-III-TOF/TOF-Massenspektrometers (Hersteller: Bruker Daltonics) bestimmt.

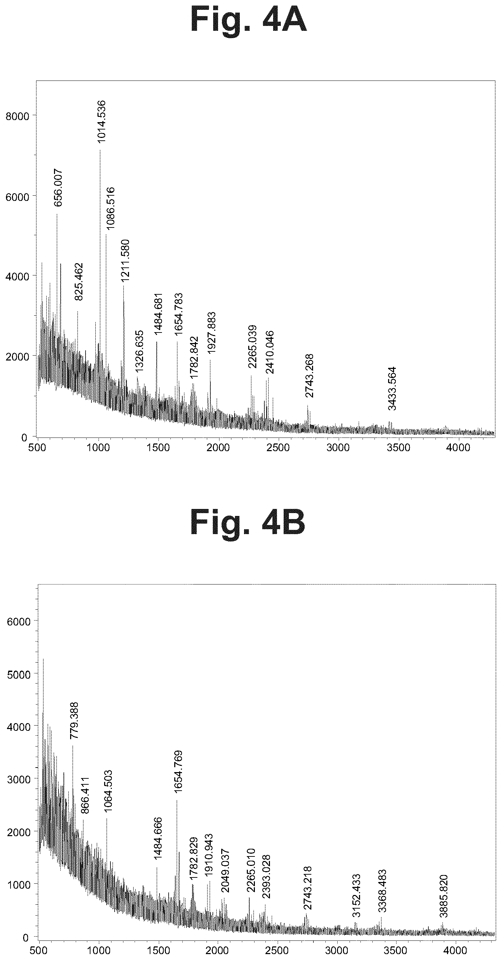

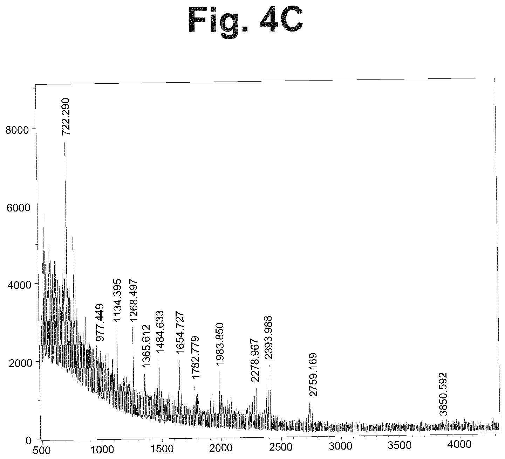

Die Figuren 4A bis 4C zeigen die entsprechenden Massenspektren bzw. Molekulargewichtsverteilungen der Kollagenhydrolysate A, B bzw. C, wobei auf der Ordinate das Molekulargewicht bzw. die Massenzahl und auf der Abszisse die Intensität aufgetragen ist. Ein Vergleich der drei Spektren zeigt, dass das Hydrolysat A die folgenden charakteristischen Peptide gemäß der Tabelle 4 umfasst, wobei die entsprechenden Peaks eine doppelte bis vierfache Intensität im Vergleich zu ihrer Umgebung aufweisen:

**Tabelle 4**

| |
|---|
| ca. 656 Da |
| ca. 825 Da |
| ca. 1.014 Da |
| ca. 1.211 Da |
| ca. 1.927 Da |
| ca. 2.410 Da |
| ca. 3.433 Da |

Insbesondere die vier Peptide zwischen 600 und 1.500 Da haben keine Entsprechungen bei den beiden kommerziellen Hydrolysaten B und C, und sind somit für das Hydrolysat A besonders charakteristisch.

### 7. Stimulation der Synthese von extrazellulären Matrixproteinen in vitro

Die Stimulation der Synthese von Kollagen (Typ I) sowie der Proteoglykane Decorin und Versican wurde *in vitro* an humanen dermalen Fibroblasten (Hautzellen) untersucht. Hierfür wurden die Zellen für 24 Stunden mit jeweils 0,5 mg/ml der Hydrolysate A, B bzw. C inkubiert und anschließend die Expression von Kollagen-RNA, Decorin-RNA und Versican-RNA mittels Realtime-PCR bestimmt und semiquantitativ ausgewertet. Decorin spielt eine wichtige Rolle bei der Bildung der Kollagenfasern in der Haut.

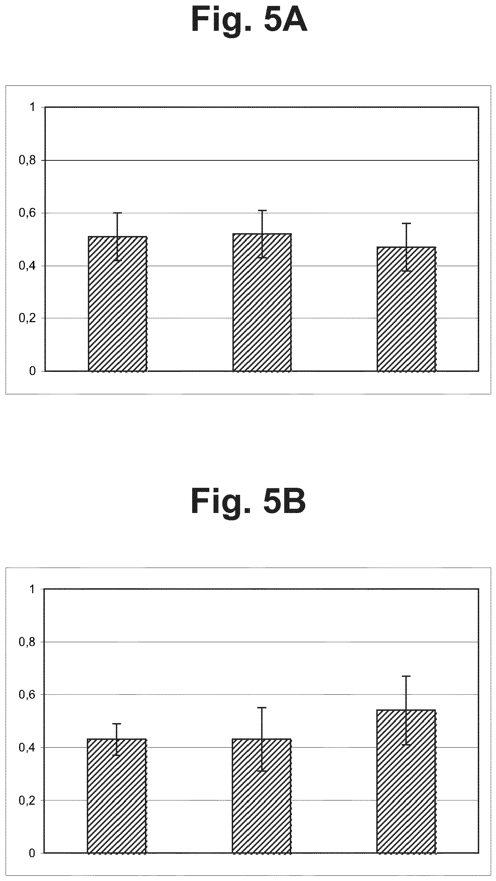

Die Ergebnisse sind als Säulendiagramme für das Hydrolysat B in der Figur 5A und für das Hydrolysat C in der Figur 5B dargestellt, wobei auf der Abszisse jeweils die RNA-Expression bei den kommerziellen Hydrolysaten B bzw. C relativ zur RNA-Expression bei dem Hydrolysat A (=1) aufgetragen ist. Die linke Säule steht jeweils für Typ-I-Kollagen, die mittlere Säule für Decorin und die rechte Säule für Versican. Es ist jeweils der Mittelwert aus mindestens 7 Messungen dargestellt sowie der Standardfehler.

Interessanterweise zeigen die Daten, dass bei allen drei Matrixproteinen eine im Vergleich zum Hydrolysat A deutlich geringere Stimulation der RNA-Synthese durch die beiden Hydrolysate B und C erfolgt, deren mittleres Molekulargewicht nur geringfügig höher ist. Die charakteristischen Peptide des Hydrolysats A scheinen somit eine entscheidende Rolle für dessen vorteilhafte Wirkung zu spielen.

### 8. Beispielrezepturen für Nahrungs(ergänzungs)mittel

Im Folgenden sind einige beispielhafte Rezepturen für die erfindungsgemäße Verwendung des Kollagenhydrolysats angegeben, die selbstverständlich in vielfältiger Weise abgewandelt werden können:

**Kapsetten (Nahrungsergänzungsmittel)**

| | |
|---|---|
| Glycerin | 53,67 Gew.% |
| **Kollagenhydrolysat** | 21,95 Gew.% |
| Gelatine | 10,08 Gew.% |
| Guarkernmehl | 6,00 Gew.% |
| Lecithin | 5,00 Gew.% |
| Citronensäure | 2,00 Gew.% |
| Aroma (Cassis) | 0,50 Gew.% |
| Orangenöl | 0,50 Gew.% |
| Acesulfam-K | 0,30 Gew.% |

**Schokolade**

| | |
|---|---|
| Kakaomasse | 51,0 Gew.% |
| Saccharose | 22,4 Gew.% |
| Kakaobutter | 16,6 Gew.% |
| **Kollagenhydrolysat** | 10,0 Gew.% |

**Getränk**

| | |
|---|---|
| Wasser | 63,00 Gew.% |
| Aloe-Vera-Konzentrat | 31,00 Gew.% |
| **Kollagenhydrolysat** | 4,00 Gew.% |
| Saccharose | 1,50 Gew.% |
| Citronensäure | 0,26 Gew.% |
| Aromen und Farbst. | 0,24 Gew.% |
| Sucralose | 0,0031 Gew.% |

## Patentansprüche

1. Kosmetische Verwendung von Kollagenhydrolysat zur Behandlung und/oder Vorbeugung von Cellulite und/oder von Dehnungsstreifen, insbesondere Schwangerschaftsstreifen, wobei das Kollagenhydrolysat ein mittleres Molekulargewicht von 1.700 bis 2.300 Da aufweist, wobei mindestens 45 Gew.% des Kollagenhydrolysats ein Molekulargewicht von weniger als 1.500 Da aufweisen, und wobei das Kollagenhydrolysat für eine orale Verabreichung vorgesehen ist.

2. Verwendung nach Anspruch 1, wobei das Kollagenhydrolysat ein Peptid zwischen 620 und 690 Da, ein Peptid zwischen 790 und 860 Da, ein Peptid zwischen 980 und 1.050 Da und ein Peptid zwischen 1.175 und 1.245 Da umfasst.

3. Verwendung nach Anspruch 1, wobei das Kollagenhydrolysat einen Anteil an Hydroxyprolin von 12 Gew.% oder mehr aufweist.

4. Verwendung nach einem der vorangehenden Ansprüche, wobei das Kollagenhydrolysat durch enzymatische Hydrolyse von Gelatine hergestellt ist.

5. Verwendung nach Anspruch 4, wobei das Kollagenhydrolysat durch die nacheinander folgende Einwirkung von mindestens zwei Endoproteasen mit einer unterschiedlichen Spezifität, insbesondere von mindestens zwei verschiedenen Metalloproteasen und/oder Serinproteasen, hergestellt ist.

6. Verwendung nach einem der vorangehenden Ansprüche, wobei mindestens 50% der N-terminalen Aminosäuren des Kollagenhydrolysats hydrophobe Aminosäuren sind, insbesondere Alanin, Leucin und Isoleucin.

7. Verwendung nach einem der Ansprüche 1 bis 6, wobei das Kollagenhydrolysat ein Nahrungsergänzungsmittel ist, und insbesondere in Form von Tabletten, Kapseln, Dragees, Pastillen, Sachets, eines Gels oder einer Lösung vorliegt.

8. Verwendung nach einem der Ansprüche 1 bis 7, wobei eine tägliche Aufnahme von ca. 1,5 bis 5 g, bevorzugt ca. 2 bis 3 g, weiter bevorzugt ca. 2,3 bis 2,7 g des Kollagenhydrolysats vorgesehen ist.

9. Verwendung nach einem der vorangehenden Ansprüche, wobei das Kollagenhydrolysat mit einem oder mehreren weiteren Wirkstoffen kombiniert ist, die ausgewählt sind aus Vitaminen, Mineralien, Omega-3-Fettsäuren, Omega-6-Fettsäuren, Omega-9-Fettsäuren, Biotin, Lutein, Lycopin, Coffein, Glucosamin, Chondroitin, Hyaluronsäure, Folsäure, Aminosäuren, Ubichinon-10, Superoxid-Dismutase sowie Pflanzenextrakten aus Hagebutten, Zitronenverbene oder Grüntee.

10. Verwendung nach einem der vorangehenden Ansprüche, wobei das Kollagenhydrolysat für eine Verabreichung an Frauen im Alter von über 50 Jahren vorgesehen ist, insbesondere an Frauen in der Postmenopause.

## Claims

1. Cosmetic use of collagen hydrolysate for treating and/or preventing cellulite and/or stretch marks, in particular pregnancy-related stretch marks, wherein the collagen hydrolysate has an average molecular weight of 1,700 to 2,300 Da, wherein at least 45% by weight of the collagen hydrolysate have a molecular weight of less than 1,500 Da, and wherein the collagen hydrolysate is provided for oral administration.

2. Use according to claim 1, the collagen hydrolysate comprising a peptide of between 620 Da and 690 Da, a peptide of between 790 Da and 860 Da, a peptide of between 980 Da and 1,050 Da and a peptide of between 1,175 Da and 1,245 Da.

3. Use according to claim 1, the collagen hydrolysate having a hydroxyproline content of 12% by weight or more.

4. Use according to one of the preceding claims, wherein the collagen hydrolysate is manufactured by the enzymatic hydrolysis of gelatine.

5. Use according to claim 4, wherein the collagen hydrolysate is produced through the sequential action of at least two endoproteases with different specificity, in particular at least two different metalloproteases and/or serine proteases.

6. Use according to one of the preceding claims, wherein at least 50% of the N-terminal amino acids of the collagen hydrolysate are hydrophobic amino acids, in particular alanine, leucine and isoleucine.

7. Use according to one of claims 1 to 6, wherein the collagen hydrolysate is a nutritional supplement and is present, in particular, in the form of tablets, capsules, sugar-coated pills, pastilles, sachets, a gel or a solution.

8. Use according to one of claims 1 to 7, wherein a daily intake of approximately 1.5 g to 5 g, preferably approximately 2 g to 3 g, more preferably approximately 2.3 g to 2.7 g of the collagen hydrolysate is provided.

9. Use according to one of the preceding claims, wherein the collagen hydrolysate is combined with one or more further active ingredients which are selected from vitamins, minerals, omega-3 fatty acids, omega-6 fatty acids, omega-9 fatty acids, biotin, lutein, lycopene, caffeine, glucosamine, chondroitin, hyaluronan, folic acid, amino acids, ubiquinone-10, superoxide dismutase and plant extracts from rose hips, lemon verbena or green tea.

10. Use according to one of the preceding claims, wherein the collagen hydrolysate is provided for administration to women aged over 50 years, in particular women in the postmenopause.

## Revendications

1. Utilisation cosmétique d'hydrolysat de collagène pour le traitement et/ou la prévention de cellulite et/ou de vergetures, en particulier de vergetures de grossesse, dans laquelle l'hydrolysat de collagène présente un poids moléculaire moyen de 1 700 à 2 300 Da, dans laquelle au moins 45 % en poids de l'hydrolysat de collagène présentent un poids moléculaire de moins de 1 500 Da, et dans laquelle l'hydrolysat de collagène est prévu pour une administration orale.

2. Utilisation selon la revendication 1, dans laquelle l'hydrolysat de collagène comporte un peptide entre 620 et 690 Da, un peptide entre 790 et 860 Da, un peptide entre 980 et 1 050 Da et un peptide entre 1 175 et 1 245 Da.

3. Utilisation selon la revendication 1, dans laquelle l'hydrolysat de collagène présente une proportion en hydroxyproline de 12 % en poids ou plus.

4. Utilisation selon l'une quelconque des revendications précédentes, dans laquelle l'hydrolysat de collagène est fabriqué par hydrolyse enzymatique de gélatine.

5. Utilisation selon la revendication 4, dans laquelle l'hydrolysat de collagène est fabriqué par l'action consécutive d'au moins deux endoprotéases avec une spécificité différente, en particulier d'au moins deux différentes métalloprotéases et/ou sérine protéases.

6. Utilisation selon l'une quelconque des revendications précédentes, dans laquelle au moins 50 % des acides aminés N-terminaux de l'hydrolysat de collagène sont des acides aminés hydrophobes, en particulier l'alanine, la leucine et l'isoleucine.

7. Utilisation selon l'une quelconque des revendications 1 à 6, dans laquelle l'hydrolysat de collagène est un complément alimentaire, et en particulier se présente sous la forme de tablettes, capsules, dragées, pastilles, sachets, d'un gel ou d'une solution.

8. Utilisation selon l'une quelconque des revendications 1 à 7, dans laquelle une prise quotidienne d'environ 1,5 à 5 g, de préférence d'environ 2 à 3 g, encore plus préférentiellement d'environ 2,3 à 2,7 g de l'hydrolysat de collagène, est prévue.

9. Utilisation selon l'une quelconque des revendications précédentes, dans laquelle l'hydrolysat de collagène est combiné à une ou plusieurs autres substances qui sont sélectionnées parmi les vitamines, les minéraux, les acides gras oméga 3, les acides gras oméga 6, les acides gras oméga 9, la biotine, la lutéine, le lycopène, la caféine, la glucosamine, la chondroïtine, l'acide hyaluronique, l'acide folique, les acides aminés, l'ubiquinone 10, la superoxyde dismutase ainsi que des extraits de plante d'églantier, de verveine citronnée ou de thé vert.

10. Utilisation selon l'une quelconque des revendications précédentes, dans laquelle l'hydrolysat de collagène est prévu pour une administration aux femmes de plus de 50 ans, en particulier aux femmes en postménopause.
